(19) **Europäisches Patentamt / European Patent Office / Office européen des brevets**

(11) **EP 2 469 273 A1**

(12) # DEMANDE DE BREVET EUROPEEN

(43) Date de publication:
**27.06.2012 Bulletin 2012/26**

(51) Int Cl.:
***G01N 27/416*** *(2006.01)*      ***G01N 33/18*** *(2006.01)*

(21) Numéro de dépôt: **11194786.7**

(22) Date de dépôt: **21.12.2011**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **21.12.2010 FR 1060917**

(71) Demandeur: **Total Raffinage Marketing
92800 Puteaux (FR)**

(72) Inventeurs:
• **Basset, Alexandre
44600 Saint-Nazaire (FR)**
• **Bolliet, Christophe
69003 Lyon (FR)**
• **Festy, Thierry
76790 Etretat (FR)**

(74) Mandataire: **Radault, Gabrielle et al
Cabinet Jolly
54, rue de Clichy
75009 Paris (FR)**

(54) **Procédé et dispositif de détection des dysfonctionnements de strippers d'eaux de procédé**

(57) Un procédé d'estimation de la concentration en ammoniaque d'une eau de procédé issue d'une unité de raffinage d'hydrocarbures contenant des acides organiques, comprenant :
- recevoir (200, 203, 205) des valeurs de mesure du pH (pH), de la température (T) et de la conductivité électrique ($C_{e1}$) de l'eau de procédé, et
- calculer (201, 202, 204) une estimation de la concentration en ammoniaque ($C_{NH3}$) de l'eau de procédé en fonction des valeurs de mesure reçues, par application d'au moins une formule polynômiale.

EP 2 469 273 A1

# Description

Procédé et dispositif de détection des dysfonctionnements de strippers d'eaux de procédé. La présente invention concerne un procédé de détermination de la teneur en ammoniaque d'une eau de procédé issue d'une unité de raffinage d'hydrocarbures.

Les eaux de procédé en raffinerie sont de sources multiples. Celles-ci peuvent notamment provenir d'effluents issus de strippers d'eau. Les strippers traitent les eaux de procédé dans des colonnes à plateaux et/ou à garnissage, à l'aide d'un flux de vapeur d'eau à contre-courant. Lorsque des strippers d'eau traitent des effluents issus d'unités produisant du sulfure d'ammonium $NH_4SH$, par exemple issus d'unités FCC (Fluid Catalytic Cracking), DGO (Désulfuration de Gas Oil), DHC (Distillate HydroCracking), HDT (hydrotraitement), viscoréducteur, etc., ils permettent la séparation de l'hydrogène sulfuré et de l'ammoniac, ainsi que des composés organiques qui sont collectés en pied de séparateur puis entraînés vers une unité de traitement des eaux résiduaires, ou renvoyées vers un dessaleur. Ces composés organiques sont généralement de petites molécules ayant des fonctions acide carboxylique (par exemple : acide benzoïque, acide acétique, acide formique, acide thiophène-2-carboxylique) ou hydroxyle aromatique (par exemple : phénol, o-crésol, 1-hydroxynaphthalène).

Le pilotage des strippers d'eaux de procédé s'effectue notamment par réglage du débit de vapeur de stripage dans une colonne de stripage en fonction de la concentration en espèces chimiques dans les eaux collectées en pied de ladite colonne. La concentration en ammoniaque dans les eaux de stripage est une donnée importante, particulièrement lorsqu'elles sont renvoyées au dessaleur. La concentration en ammoniaque dans les eaux collectées en pied de stripper augmente lorsque le débit de vapeur de stripage diminue.

D'un autre côté, un débit de vapeur trop élevé dans un stripper d'eaux de procédé, tout en permettant de diminuer la concentration en ammoniaque dans les eaux collectées en pied de stripper, entrainera une surconsommation d'énergie et des rejets aqueux abondants risquant de saturer les unités de traitement des eaux résiduaires.

Ainsi, l'optimisation du débit de vapeur en pied de colonne de stripper permet de limiter les rejets aqueux et d'éviter l'engorgement des unités de traitement des eaux.

Idéalement, la teneur en ammoniaque d'une eau collectée en fond de stripper d'eaux de procédé est nulle. Réellement, elle est habituellement fixée entre 30 et 50 ppm.

Habituellement, le contrôle du bon fonctionnement d'un stripper d'eaux de procédé s'effectue par des prises d'échantillons et par leur analyse en laboratoire de la concentration en ammoniaque selon la norme NF T90-015-1 ou équivalent. De fait, les méthodes utilisées au laboratoire ne permettent pas une mesure en continu, et les contraintes opérationnelles et liées aux analyses ne permettent pas d'effectuer plus de deux prélèvements par jour. Les résultats d'analyses sont habituellement obtenus après plusieurs dizaines de minutes, ce qui ne permet pas d'agir rapidement sur le stripper. En effet, le temps que la mesure de la concentration en ammoniaque soit effectuée, la concentration réelle en ammoniaque dans la colonne de stripage a le temps de dériver, et soit (i) le débit de vapeur est insuffisant et la concentration en ammoniaque est non conforme, soit (ii) le débit de vapeur est excessif, ce qui engendre un surcoût et une dépense énergétique accrue, une concentration en ammoniaque inférieure à la norme, et une dilution importante des rejets aqueux envoyés à la station de traitement des eaux.

Ainsi, la mesure de la concentration en ammoniaque directement sur le stripper d'eau, ou sur une conduite en sortie de stripper permettrait d'améliorer la gestion du débit de vapeur sur le stripper.

Il a été envisagé d'utiliser des analyseurs en ligne commerciaux de la concentration en ammoniaque, impliquant généralement des électrodes ioniques spécifiques à l'ammoniaque ou des méthodes photométriques. Ils souffrent de problèmes de fiabilité dans les conditions d'utilisation habituelle, sont coûteux et ont un délai de réponse important. Dans le cadre de la présente application, les analyseurs en ligne d'ammoniaque utilisés sur les eaux de rejet sont soumis à un fort encrassement les rendant inopérants pour une utilisation sur des strippers d'eaux : ces appareils sont relativement fragiles et couteux, quelle que soit la technologie employée (colorimétrie, électrodes sélectives d'ions). Le délai de réponse n'est pas nécessairement court, et l'adaptation des appareillages en milieu ATEX est coûteux et nécessite une maintenance importante.

Une eau de procédé issue de FCC (Fluid Catalytic Cracking = Craquage Catalytique en lit Fluidisé) ou de DGO (Désulfuration de Gas Oil) ou autre (DHC, HDT, viscoréducteur, etc.) a typiquement la composition suivante :

Phénol et dérivés : 0-300 ppm
Acides organiques : 0-100 ppm
Ammoniaque : 4000 ppm
Hydrogène sulfuré : 5000 ppm

Toutes ces espèces ont une influence sur le pH et/ou la conductivité de l'eau.

Il existe donc un besoin pour une estimation en continu de la concentration en ammoniaque des eaux collectées en pied de strippers d'eaux de procédé.

A cette fin, et selon un premier aspect de l'invention, la demanderesse a trouvé un procédé de détermination de la concentration en ammoniaque d'une eau de procédé issue d'une unité de raffinage d'hydrocarbures contenant des acides organiques, comprenant :

- recevoir des valeurs de mesure de pH, de température et de conductivité électrique de cette eau de procédé,

- calculer une estimation de la concentration en ammoniaque de l'eau de procédé en fonction des valeurs de mesure reçues, par application d'au moins une formule polynômiale.

Ce procédé peut permettre une estimation en continu de la concentration en ammoniaque.

Ce procédé peut en outre être facilement mis en oeuvre dans des moyens de traitement, les formules polynômiales étant faciles à programmer et rapides à exécuter. Les paramètres de pH, température et conductivité sont relativement faciles à mesurer.

Un tel analyseur multi-paramètres (pH, température, conductivité), de conception relativement simple, peut permettre d'estimer de façon continue la teneur en ammoniaque de l'eau en sortie d'un stripper, et donc d'évaluer l'efficacité de ce stripper et éventuellement d'avertir les opérateurs de tout dysfonctionnement à partir d'un seuil d'alarme prédéfini.

Ce procédé, impliquant une mesure de la conductivité, peut permettre d'éviter les problèmes liés à un éventuel encrassement prématuré d'une sonde de mesure du type électrode ionique spécifique à l'ammoniaque.

L'application de l'invention selon son premier aspect permet de protéger les unités de traitement des eaux, notamment lorsqu'elle conduit à une diminution du pH, car cela permet une amélioration de la DCO (Demande Chimique en Oxygène) de l'eau à traiter, en raison de la plus faible présence d'acides naphténiques. L'invention est applicable de préférence à une température comprise entre 15 et 50°C, ces deux bornes incluses.

L'invention est applicable de préférence à un pH compris entre 7 et 10,3 ; ces deux valeurs incluses.

L'invention est applicable de préférence à une concentration en ammoniaque de l'eau de procédé comprise entre 0 et 400 mg/L, ces deux bornes incluses.

L'invention est applicable de préférence à une concentration en acides organiques supérieure à 0 ppm et inférieure ou égale à 400 ppm.

Avantageusement, on pourra prévoir :

- d'obtenir un jeu d'au moins une valeur de coefficient par application d'un polynôme ayant pour indéterminée la valeur de température, et/ou
- d'obtenir un ensemble d'au moins une valeur de coefficient par application d'un polynôme ayant pour indéterminée la valeur de pH,
- de calculer l'estimation de concentration en fonction de la valeur de conductivité, et du jeu et de l'ensemble obtenus.

Les polynômes peuvent ainsi être avec une seule indéterminée chacun.

Avantageusement, le jeu ou l'ensemble obtenu peuvent servir pour l'obtention respectivement de l'ensemble ou du jeu.

Avantageusement, les coefficients du polynôme ayant pour indéterminée la température sont issus de l'ensemble d'au moins une valeur de coefficient, cet ensemble étant obtenu à partir de la valeur de pH reçue.

Avantageusement, les coefficients du polynôme ayant pour indéterminée le pH sont lus dans une mémoire. Ces coefficients peuvent par exemple être obtenus à partir de données d'étalonnage.

Avantageusement, la mémoire est structurée de façon à stocker plusieurs jeux (d'au moins un coefficient chacun), chaque jeu de coefficient étant associé à une plage de pH. Le procédé peut ainsi comprendre une étape de comparaison de la valeur de pH reçue à un (ou plusieurs) seuil, et, en fonction du résultat de la comparaison, une étape de sélection d'un jeu de coefficients de polynôme.

Ainsi, la formule polynômiale ayant pour indéterminée le pH peut être choisie en fonction de la valeur de pH. Ceci peut permettre une meilleure adéquation avec des valeurs d'étalonnage.

La ou les formules polynômiales peuvent être relativement simples, d'ordre 1 ou 2 par exemple, ou bien d'ordres supérieurs.

La valeur estimée de concentration en ammoniaque peut être utilisée pour piloter des moyens de régulation du débit de vapeur dans un stripper d'eau. Le procédé peut comprendre une étape de génération d'un signal de commande de ces moyens, en fonction de la valeur estimée de concentration en ammoniaque, et de transmission de ce signal vers ces moyens.

Selon un second aspect, l'invention concerne un logiciel permettant de déterminer la concentration théorique en ammoniaque d'une eau de procédé contenant des acides organiques. Il est ainsi proposé un produit programme d'ordinateur pour estimer la concentration en ammoniaque d'une eau de procédé issue d'une unité de raffinage d'hydrocarbures contenant des acides organiques, comprenant des instructions pour effectuer les étapes du procédé décrit ci-dessus lorsqu'il est exécuté par des moyens de traitement de type processeur.

Selon un troisième aspect, l'invention concerne un dispositif de mesure de la concentration en ammoniaque dans une eau de procédé contenant des acides organiques, comprenant

- des moyens de réception de valeurs de mesure du pH, de la température et de la conductivité électrique de l'eau de procédé,
- des moyens de traitement agencés pour calculer une estimation de la concentration en ammoniaque de l'eau de procédé en fonction des valeurs de mesure reçues, par application d'au moins une formule polynômiale.

Ce dispositif peut ainsi permettre de mettre en oeuvre le procédé décrit ci-dessus. En particulier le dispositif peut être agencé de façon à générer un signal de commande, et être disposé de façon à pouvoir commander, via le signal de commande généré, des moyens de régulation du débit de vapeur dans un stripper d'eau.

Le dispositif peut par exemple comprendre ou être intégré dans un processeur, par exemple un microcontrôleur, un microprocesseur ou un DSP (de l'anglais (« Digital Signal Processor »).

Les moyens de réception peuvent par exemple comprendre une pin d'entrée, un port d'entrée ou autre.

Les moyens de traitement peuvent par exemple comprendre un coeur de processeur ou CPU (de l'anglais « Central Processing Unit »).

Avantageusement, et selon son troisième aspect, l'invention concerne un ordinateur comprenant le logiciel commande des moyens de régulation du débit de vapeur dans un stripper d'eau.

Il est en outre proposé un système d'estimation de la concentration en ammoniaque d'une eau de procédé issue d'une unité de raffinage d'hydrocarbures contenant des acides organiques, comprenant :

- un dispositif d'estimation tel que décrit ci-dessus,
- au moins une sonde de mesure de température en communication avec le dispositif d'estimation,
- au moins une sonde de mesure de conductivité en communication avec le dispositif d'estimation,
- au moins une sonde de mesure de pH en communication avec le dispositif d'estimation,
- des moyens de transmission et/ou d'affichage de la valeur calculée par les moyen de traitement du dispositif d'estimation.

Il est en outre proposé une unité de stripage d'eau de procédé comprenant :

- une colonne de stripage alimentée par un flux aqueux comprenant de l'ammoniaque, des acides organiques, de l'hydrogène sulfuré ($H_2S$), dans laquelle le flux aqueux est balayé (strippé) par un flux de vapeur pour produire en tête un flux de vapeur contenant une majorité de l'ammoniaque et de l'$H_2S$, et en fond un flux aqueux contenant une majorité des acides organiques,
- un dispositif ou un système tel que décrit ci-dessus,
- une sonde de mesure de conductivité disposée de façon à mesurer la conductivité du flux aqueux de fond de colonne.

L'unité de stripage d'eaux de procédé peut en outre comprendre des moyens de mesure du pH du flux aqueux de fond de colonne, par exemple un pH-mètre disposé en fond de colonne.

L'unité de stripage d'eaux de procédé peut en outre comprendre des moyens de mesure de la température du flux aqueux de fond de colonne, par exemple une sonde de mesure disposée en fond de colonne.

Les moyens de mesure du pH du flux aqueux de fond de colonne et/ou de la température du flux aqueux de fond de colonne sont connectés à un système d'acquisition des données générées par lesdits moyens de mesure.

La figure 1 représente une unité de strippage d'eau de procédé selon un mode de réalisation de l'invention.

La figure 2 est un organigramme d'un exemple de procédé selon un mode de réalisation de l'invention.

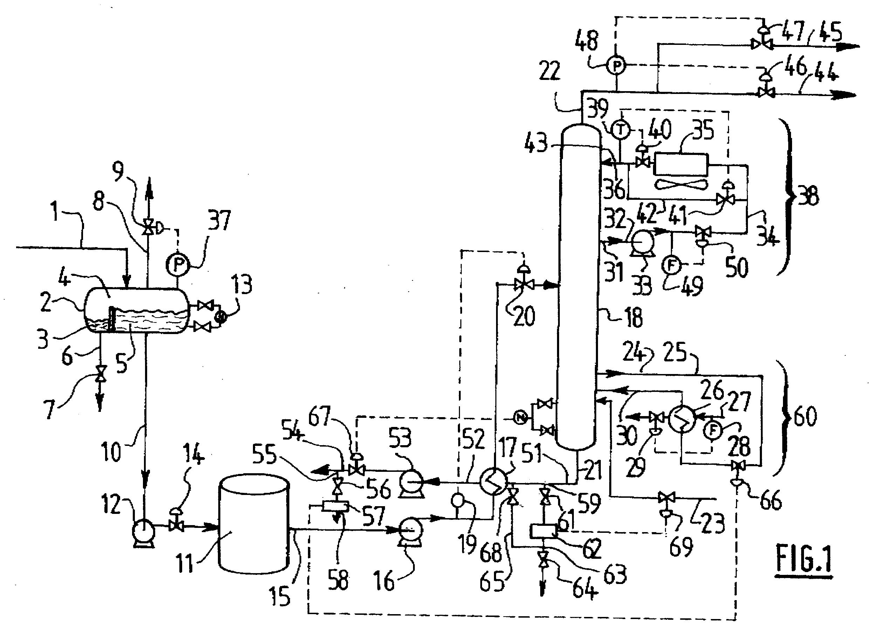

Sur la figure 1, une eau de procédé à traiter (1) est introduite dans un ballon de séparation (2) agencé de façon à permettre une séparation primaire entre (i) les hydrocarbures liquides résiduels (3) ayant un densité inférieure à la phase aqueuse, qui sont entraînés dans la conduite (6) munie d'une vanne de purge (7), (ii) les composés volatils (4), qui sont entraînés dans la conduite (8) munie d'une vanne régulée (9) par un contrôleur de pression (37) sur le ballon (2), et (iii) la phase aqueuse à traiter (5), entraînée dans une conduite (10). La phase aqueuse à traiter (5) est envoyée dans une capacité (11) au moyen d'une pompe (12). La vidange du ballon de séparation (2) au niveau de la conduite (5) transportant la phase aqueuse à traiter (5), est commandée par un contrôleur de niveau (13) disposé sur le ballon (2). Le contrôleur de niveau (13) commande une vanne (14) positionnée entre la pompe (12) et la capacité (11).

La phase aqueuse à traiter (5) est soutirée de la capacité (11) par une conduite (15) au moyen d'une pompe (16), réchauffée dans un échangeur de chaleur (17), puis est introduite sensiblement vers le milieu d'une colonne de stripage (18). La colonne de stripage (18) est habituellement équipée d'une dizaine de plateaux théoriques. Le débit de la phase aqueuse à traiter (5) est géré par un contrôleur de débit (19) qui régule l'ouverture d'une vanne (20) placée à l'entrée de la colonne de stripage.

La phase aqueuse à traiter (5) est séparée dans la colonne de stripage (18) en (i) une eau de procédé (21), appauvrie en $H_2S$ et ammoniaque, collectée en fond de colonne (18), et (ii) un gaz enrichi en $H_2S$ et ammoniac (22), collecté en tête de colonne (18).

La colonne de stripage (18) est équipée d'une alimentation en vapeur (23) en bas de colonne. La colonne (18) peut également être équipée d'un rebouilleur de fond de colonne (60) qui consiste en une conduite de prélèvement (24) qui soutire une fraction liquide (25) sur un plateau vers le bas de la colonne (18). La fraction liquide (25) est envoyée dans un échangeur thermique (26) pour y être réchauffée par un fluide chaud (27), habituellement un flux de vapeur, pour produire une fraction réchauffée (30) au moins partiellement vaporisée. Le débit de fluide chaud (27) est régulé par un contrôleur de débit (28) commandant l'ouverture d'une vanne (29) placée en aval de l'échangeur (26). La fraction réchauffée (30) est introduite en dessous du plateau comportant la conduite de prélèvement (24). De préférence, on équipera une colonne de stripage (18) d'un rebouilleur de fond de colonne (60) tel que décrit ci-dessus, plutôt que d'une alimentation en vapeur (23). Toutefois, l'alimentation en vapeur (23) sera nécessaire en cas de nettoyage du rebouilleur de fond de colonne (60).

La colonne de stripage (18) est avantageusement équipée d'un reflux circulant (38) ou d'un ballon de reflux (non représenté). Ledit reflux (38) comprend une conduite de prélèvement (31) située au dessus d'un plateau qui est lui-même au dessus de la zone d'introduction de la phase aqueuse à traiter (5) de la colonne (18). Ladite conduite de prélèvement (31) prélève un flux (32) au moyen d'une pompe (33) qui l'achemine par une conduite (34) vers un échangeur thermique (35), ici un aéro-réfrigérant, qui produit un flux refroidi (36) injecté par une conduite (43) au moins un plateau au dessus de la zone de prélèvement (31). La température du flux refroidi (36) est régulée par des moyens de mesure de température (39), comprenant par exemple un processeur et une sonde de température, qui commandent (i) l'ouverture d'une vanne (40) située immédiatement à la sortie de l'échangeur (35), et (ii) l'ouverture d'une vanne (41) située sur une conduite de dérivation (42). Une extrémité de ladite conduite (42) est connectée à la conduite (34) et l'autre extrémité est connectée à la conduite (43) entre la vanne (40) et les moyens de mesure de température (39). Le débit du flux (32) est régulé par des moyens de contrôle de débit (49) positionnés sur la conduite (34) en sortie de pompe (33). Les moyens de contrôle de débit (49) commandent l'ouverture d'une vanne (50) située en aval sur la conduite (34).

Le gaz enrichi en $H_2S$ et ammoniac (22), collecté en tête de colonne de stripage (18), est envoyé vers une unité de récupération du soufre, par exemple de type Claus (SRU = Sulfur Recovery Unit), par une conduite (44). La conduite (44) est équipée d'une vanne (46) et d'une conduite de dérivation (45) vers une torche acide, également équipée d'une vanne (47). Des moyens de mesure de pression (48), comprenant un processeur et un capteur de pression monté sur la conduite (44) à proximité de la tête de la colonne (18), contrôlent l'ouverture des vannes (46) et (47). Lorsque la pression est trop forte, Les moyens de mesure de pression (48) envoient un signal pour permettre l'ouverture de la vanne (47). Dans ce cas, les gaz sont envoyés vers un réseau torche. La conduite (45) est connectée sur la conduite (44) entre la vanne (46) et les moyens de mesure de pression (48).

L'eau de procédé (21) soutirée en fond de colonne (18) est envoyée par une conduite (51) dans l'échangeur (17) pour produire une eau de procédé refroidie (52). Cette dernière est évacuée par une pompe (53) puis par une conduite (54). Le niveau liquide en fond de colonne (18) est régulé par des moyens de contrôle de niveau positionnés en dessous de l'alimentation en vapeur (23) ou du rebouilleur de fond de colonne (60). Les moyens de contrôle de niveau commandent l'ouverture d'une vanne (67) placée en aval de la pompe (53).

La conduite (54) est munie d'une conduite de dérivation (55) isolable au moyen d'une vanne (56), qui permet d'envoyer une partie de l'eau de procédé refroidie (52) vers des premiers moyens de mesure de la conductivité, de la température et du pH (57), équipés d'une purge (58). Ces premiers moyens (57) comprennent une portion de conduit équipée de la purge (58), un capteur de conductivité pour mesurer la conductivité du fluide dans cette portion de conduit, à deux électrodes ou davantage, une sonde de température pour mesurer la température dans cette portion de conduit, un pH-mètre pour mesurer le pH du fluide dans la portion de conduit, et un processeur en communication avec le capteur de conductivité, la sonde et le pH-mètre.

Alternativement ou en complément, la conduite (51) est équipée d'une conduite de dérivation (59) isolable au moyen d'une vanne (61), qui permet d'envoyer une partie de l'eau de procédé (21) vers des seconds moyens de mesure de la conductivité, de la température et du pH (62), équipés d'une conduite de purge (63), elle-même équipée d'une vanne de purge (64).

Ces deuxièmes moyens (62) comprennent une portion de conduit avec la conduite de purge (63), un capteur de conductivité pour mesurer la conductivité du fluide dans cette portion de conduit, à deux électrodes ou davantage, une sonde température pour mesurer la température du fluide dans cette portion de conduit, un pH-mètre pour mesurer le pH du fluide dans cette portion de conduit, et un processeur en communication avec le capteur de conductivité, la sonde et le pH-mètre.

Selon un mode de réalisation préféré, une conduite de dérivation (65) comprenant une vanne (68) est connectée à une extrémité à la conduite (51) entre la conduite de dérivation (59) et l'échangeur (17), et à l'autre extrémité à la conduite de purge (63) entre les seconds moyens de mesure de la conductivité, de la température et du pH (62), et la vanne de purge (64).

Selon un mode de réalisation représenté au moyen de pointillés entre les éléments 62 et 69, les seconds moyens de mesure de la conductivité, de la température et du pH (62), et plus précisément le processeur de ces seconds moyens, commande(nt) une vanne (69) qui régule le débit de l'alimentation en vapeur (23).

Selon un mode de réalisation non représenté, le processeur des seconds moyens de mesure de la conductivité, de la température et du pH (62), commande une vanne (66), positionnée sur la conduite de prélèvement (24), afin de réguler le débit de fraction réchauffée (30) issue du rebouilleur de fond de colonne (60).

Selon un mode de réalisation représenté au moyen de pointillés entre les éléments 57 et 66, le processeur des premiers moyens de mesure de la conductivité, de la température et du pH (57), commande la vanne (66), positionnée sur la conduite de prélèvement (24), afin de réguler le débit de fraction réchauffée (30) issue du rebouilleur de fond de colonne (60).

Selon un mode de réalisation non représenté, le processeur des premiers moyens de mesure de la conductivité, de la température et du pH (57), commande la vanne (69), qui régule le débit de l'alimentation en vapeur (23).

Un stripper d'eaux de procédé peut comporter un ou plusieurs moyens de mesure de la conductivité, de la température et du pH (57) et/ou (62). Ces derniers peuvent intégrer un logiciel de calcul de la concentration théorique en ammoniaque, ou bien être en communication avec un processeur non représenté et programmé pour effectuer ce calcul.

Les processeurs des moyens 57, 62 peuvent être distincts ou non. Ces processeurs peuvent être agencés pour exécuter le procédé décrit ci-dessous, ou bien pour transmettre les valeurs mesurées de conductivité, température et pH vers un autre processeur.

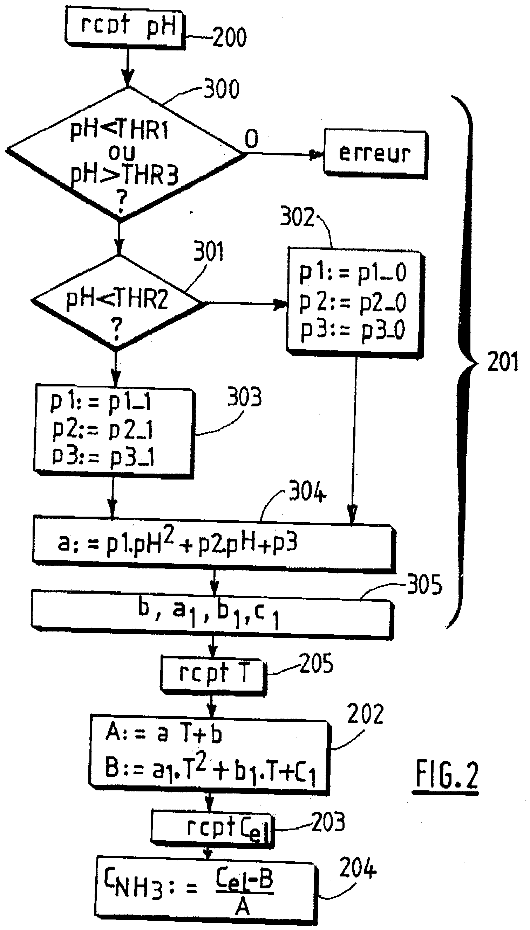

La figure 2 est un organigramme d'un exemple de procédé exécutable par le processeur 57, le processeur 62 et/ou par un processeur non représenté sur la figure 1.

Une valeur de mesure de pH est reçue au cours d'une étape (200). Puis, au cours d'une étape (201), sont calculées les valeurs de cinq coefficients $a$, $b$, $a_1$, $b_1$, $c_1$, par application d'une formule polynômiale avec pour indéterminée la valeur de pH reçue.

Plus précisément, pour chacun de ces coefficients $a$, $b$, $a_1$, $b_1$, $c_1$, sont effectuées les étapes décrites ci-dessous. Sur la figure 2, afin de ne pas alourdir inutilement la figure, ces étapes en sont détaillées que pour le coefficient a.

La valeur de pH reçue est comparée à plusieurs seuils de pH, afin d'estimer dans quelle plage de valeurs de pH, parmi une pluralité de plages prédéterminées, se trouve la valeur de pH reçue. On pourra par exemple prévoir de comparer la valeur de pH reçue à 7, à 9 et à 10,3. S'il s'avère que la valeur reçue est inférieure à 7 ou supérieure à 10,3, un signal d'erreur pourra être émis.

Ces comparaisons sont représentées par les tests 300, 301 de la figure 2.

Les seuils peuvent être identiques pour tous les coefficients $a$, $b$, $a_1$, $b_1$, $c_1$, auquel cas on pourra n'effectuer ces étapes de comparaison qu'une seule fois. Mais avantageusement, les seuils ne sont pas forcements identiques d'un coefficient à l'autre et les étapes de comparaison sont effectuées pour chaque, ou pour au moins deux, coefficient.

Puis, une fois déterminée la plage de valeurs correspondant à la valeur reçue, on choisit le jeu de coefficients de polynôme {p1, p2, p3} comme étant celui associé à la plage de valeurs de pH dans laquelle se trouve la valeur de pH reçue. Ceci est illustré par les étapes 302 et 303.

Les cinq jeux de coefficients sont ainsi lus en mémoire ce qui permet une exécution du procédé relativement rapide.

Ces coefficients de polynôme p1_0, p2_0, p3_0, p1_1, p2_1, p3_1 etc. peuvent être déterminés de façon empirique, par exemple en se basant sur des données d'étalonnage. On pourra en particulier utiliser le logiciel « OLI analyzer », développé par la société OLI systems, (Morris Plains, New Jersey, Etats-Unis), pour déterminer ces coefficients à programmer en mémoire.

Pour chaque coefficient $a$, $b$, $a_1$, $b_1$, $c_1$, on pourra prévoir une seule plage de valeurs de pH, deux, trois, quatre plages, ou même davantage.

Dans un mode de réalisation avantageux, chaque jeu de coefficients de polynôme stocké en mémoire comporte au maximum trois coefficients: l'un p1_0, p1_1, etc., éventuellement nul correspondant au terme d'ordre 2, le deuxième p2_0, p2_1, etc., pour le terme d'ordre 1, et le troisième p3_0, p3_1, etc. pour le terme d'ordre zéro. Le polynôme correspondant à ces coefficients de polynôme peut être d'ordre 1 ou 2, selon le coefficient $a$, $b$, $a_1$, $b_1$, $c_1$ et la plage de pH associée.

Une fois le jeu de coefficients de polynôme sélectionné, la valeur de coefficient $a$, $b$, $a_1$, $b_1$, ou $c_1$ est calculée en appliquant le polynôme d'ordre 1 ou 2 correspondant à ces coefficient p1, p2, p3. Ceci est représenté par l'étape 304 sur la figure 2.

L'étape 305 de la figure 2 représente l'obtention des quatre autres coefficients $b$, $a_1$, $b_1$, $c_1$, selon des procédés similaires. On a ainsi calculé un ensemble de cinq coefficients $a$, $b$, $a_1$, $b_1$, $c_1$ en appliquant cinq formules polynômiales respectives, d'ordre 1 ou 2, avec la valeur de pH reçue comme indéterminé.

Puis une valeur de température est reçue au cours de l'étape 205.

Au cours de l'étape 202, est calculé un jeu de deux valeurs A, B, en appliquant deux polynômes ayant la valeur de température reçue comme indéterminée. Les coefficients de ces polynômes sont les valeurs $a$, $b$, $a_1$, $b_1$, $c_1$ déterminées au COUTS de l'étape 201.

La valeur A est obtenue par application d'un polynôme d'ordre 1, et B est obtenue par application d'un polynôme d'ordre 2.

Puis, lors d'une étape 203, une valeur de conductivité électrique $C_{e1}$ est reçue. La conductivité peut avoir été mesurée en faisant passer un courant alternatif très basse tension entre deux électrodes.

Une valeur estimée de la concentration en ammoniaque $C_{NH3}$ est calculée au cours d'une étape 204, à partir de la valeur de conductivité électrique $C_{c1}$ reçue et des valeurs A, B, calculées à l'étape 202, selon :

$$C_{NH3} = \frac{C_{el} - B}{A}$$

Pour revenir à la figure 1, les moyens 66 et/ou 69 sont commandés en fonction de cette estimation de la concentration en ammoniaque $C_{NH3}$.

Le modèle de calcul du procédé de la figure 2 est relativement simple à mettre en oeuvre. On a comparé les résultats obtenus à des valeurs mesurées en laboratoire pour plusieurs strippers d'eaux de raffineries. Il ressort que dans la plage de températures de 15 à 50°C, dans la plage de pH de 7 à 10,3 et pour des concentrations en ammoniaque variant de 3 à 250 mg/litre, l'écart maximal entre les valeurs mesurées suivant la méthodes NFT 90-015-1 et les valeurs calculées grâce au modèle décrit ci-dessus est de l'ordre de 10%. Cet écart est à comparer avec la répétabilité de cette méthode qui est de l'ordre de 5% au niveau de 50 mg/litre

**Revendications**

1. Procédé d'estimation de la concentration en ammoniaque d'une eau de procédé issue d'une unité de raffinage d'hydrocarbures contenant des acides organiques, comprenant :

   recevoir (200, 203, 205) des valeurs de mesure du pH (pH), de la température (T) et de la conductivité électrique ($C_{e1}$) de l'eau de procédé, et calculer (201, 202, 204) une estimation de la concentration en ammoniaque ($C_{NH3}$) de l'eau de procédé en fonction de la valeur de pH reçue, de la valeur de température reçue et de la valeur de conductivité électrique reçue, par application d'au moins une formule polynômiale.

2. Procédé selon la revendication 1, comprenant en outre, au cours de l'étape de calcul :

   (i) calculer (201) un ensemble d'au moins une valeur de coefficient ($a$, $b$, $a_1$, $b_1$, $c_1$) par application d'une formule polynômiale d'ordre 1 ou 2, l'indéterminée de ladite formule polynômiale étant la valeur de pH reçue (pH).

3. Procédé selon la revendication 2, comprenant en outre:

   comparer (200, 201) la valeur de pH reçue (pH) à un seuil (THR1, THR2, THR3), et choisir (302, 303) la formule polynômiale de l'étape (i) en fonction du résultat de la comparaison.

4. Procédé selon l'une des revendications 1 à 3, comprenant en outre, au cours de l'étape de calcul : .

   (ii) calculer (202) un jeu d'au moins une valeur de coefficient (A, B) par application d'une formule polynômiale d'ordre 1 ou 2, l'indéterminée de ladite formule polynômiale étant la valeur de température reçue (T).

5. Procédé selon la revendication 4 lorsqu'elle dépend de la 2 ou de la 3, dans lequel, au cours de l'étape (ii), les valeurs de coefficient ($a$, $b$, $a_1$, $b_1$, $c_1$) de la formule polynomiale ayant pour indéterminée la valeur de température ont été calculées au cours de l'étape (i).

6. Procédé selon l'une des revendications 4 ou 5, comprenant en outre, au cours de l'étape de calcul,

   (iii) calculer (204) l'estimation de la concentration en ammoniaque ($C_{e1}$) à partir du jeu d'au moins une valeur de coefficient (A, B) calculée à l'étape (ii) et à partir de la valeur de conductivité reçue ($C_{e1}$).

7. Procédé selon l'une des revendications 1 à 6, comprenant en outre
   générer un signal de commande de moyens de régulation (69) de débit de vapeur en fonction de l'estimation de concentration en ammoniaque calculée, transmettre le signal de commande ainsi généré vers les moyens de régulation du débit de vapeur dans un stripper d'eau.

8. Procédé selon l'une des revendications 1 à 7, dans lequel la température est comprise entre 15 et 50°C, inclus.

9. Procédé selon l'une des revendications 1 à 8, dans lequel le pH est compris entre 7 et 10,3 inclus.

10. Procédé selon l'une des revendications 1 à 9, dans lequel la concentration en ammoniaque de l'eau de procédé est comprise entre 0 et 400 mg/ L inclus.

11. Procédé selon l'une des revendications 1 à 10, dans lequel la concentration en acides organiques est supérieure à 0 ppm et inférieure ou égale à 400 ppm.

12. Produit programme d'ordinateur pour estimer la concentration en ammoniaque d'une eau de procédé issue d'une unité de raffinage d'hydrocarbures contenant des acides organiques, comprenant des instructions pour effectuer les étapes du procédé selon l'une des revendications 1 à 11 lorsqu'il est exécuté par des moyens de traitement de type processeur.

13. Dispositif d'estimation (57, 62) de la concentration en ammoniaque d'une eau de procédé issue d'une unité de raffinage d'hydrocarbures contenant des acides organiques, comprenant
    des moyens de réception de valeurs de mesure du pH, de la température et de la conductivité électrique de l'eau de procédé,
    des moyens de traitement agencés pour calculer une estimation de la concentration en ammoniaque de l'eau de procédé en fonction des valeurs de mesure reçues, par application d'au moins une formule polynômiale.

14. Système d'estimation de la concentration en ammoniaque d'une eau de procédé issue d'une unité de raffinage d'hydrocarbures contenant des acides organiques, comprenant

un dispositif d'estimation (57, 62) selon la revendication 13,
au moins une sonde de mesure de température en communication avec le dispositif d'estimation,
au moins une sonde de mesure de conductivité en communication avec le dispositif d'estimation,
au moins une sonde de mesure de pH en communication avec le dispositif d'estimation,
des moyens de transmission et/ou d'affichage de la valeur calculée par les moyen de traitement du dispositif d'estimation.

15. Unité de stripage d'eau de procédé comprenant une colonne de stripage (18) alimentée par un flux aqueux comprenant de l'ammoniaque, des acides organiques, de l'hydrogène sulfuré (H2S), dans laquelle le flux aqueux est balayé (strippé) par un flux de vapeur pour produire en tête un flux de vapeur contenant une majorité de l'ammoniaque et de l'H2S, et en fond un flux aqueux contenant une majorité des acides organiques,
un système selon la revendication 14,
dans lequel la sonde de mesure de conductivité est disposée de façon à mesurer la conductivité du flux aqueux de fond de colonne.

FIG.1

$$\text{rcpt pH} \quad \text{—}200$$

300

$$pH < THR1 \text{ ou } pH > THR3 \text{ ?}$$

O → erreur

302

$$p1 := p1\_0$$
$$p2 := p2\_0$$
$$p3 := p3\_0$$

301

$$pH < THR2 \text{ ?}$$

303

$$p1 := p1\_1$$
$$p2 := p2\_1$$
$$p3 := p3\_1$$

304

$$a := p1.pH^2 + p2.pH + p3$$

305

$$b, a_1, b_1, c_1$$

201

$$\text{rcpt T} \quad \text{—}205$$

202

$$A := a\,T + b$$
$$B := a_1.T^2 + b_1.T + c_1$$

$$\text{rcpt } C_{el} \quad \text{—}203$$

204

$$C_{NH_3} := \frac{C_{el} - B}{A}$$

FIG.2

Europäisches
Patentamt

European
Patent Office

Office européen
des brevets

**RAPPORT DE RECHERCHE EUROPEENNE**

Numéro de la demande

EP 11 19 4786

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | MARIE H MASSON ET AL: "Software sensor design based on empirical data", ECOLOGICAL MODELLING, vol. 120, no. 2-3, 13 août 1999 (1999-08-13), pages 131-139, XP055002290, ISSN: 0304-3800, DOI: 10.1016/S0304-3800(99)00097-6 | 1-14 | INV. G01N27/416 G01N33/18 |
| Y | * le document en entier * | 15 | |
| A | JP 2002 131262 A (ORGANO KK) 9 mai 2002 (2002-05-09) * abrégé * | 13 | |
| A | US 5 882 937 A (SAUER RICHARD L [US] ET AL) 16 mars 1999 (1999-03-16) * revendications 10,11 * | 1-15 | |
| A | V V SHCHERBAKOV ET AL: "Electrical Conductivity of the Ammonia-Water System", RUSSIAN JOURNAL OF INORGANIC CHEMISTRY, vol. 54, no. 2, 1 janvier 2009 (2009-01-01), pages 277-279, XP055002287, ISSN: 0036-0236, DOI: 10.1134/S0036023609020193 * figure 2 * | 13 | |
| A | EP 1 338 891 A1 (TOYO ENGINEERING CORP [JP]) 27 août 2003 (2003-08-27) * page 5, ligne 33 - ligne 43; figure 3 * | 1,13 | |
| Y | TIM ARMSTRONG, BRUCE SCOTT, KIN TAYLOR AND ART GARDNER: "Sour Water Stripping", TODAY'S REFINERY, 28 juin 1996 (1996-06-28), XP9158148, * le document en entier * | 15 | |

DOMAINES TECHNIQUES RECHERCHES (IPC)

G01N

-/--

Le présent rapport a été établi pour toutes les revendications

| Lieu de la recherche | Date d'achèvement de la recherche | Examinateur |
|---|---|---|
| Munich | 3 avril 2012 | Purdie, Douglas |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

& : membre de la même famille, document correspondant

EPO FORM 1503 03.82 (P04C02)

RAPPORT DE RECHERCHE EUROPEENNE

Numéro de la demande

EP 11 19 4786

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | MUNOZ P C ET AL: "Design and Development of a Soft-Sensor for Ammonia Degradation and Nitrite Accumulation in an Activated Sludge reactor", INDUSTRIAL ELECTRONICS AND CONTROL APPLICATIONS, 2005. ICIECA 2005. INTERNATIONAL CONFERENCE ON QUITO, ECUADOR 30-02 NOV. 2005, PISCATAWAY, NJ, USA,IEEE, 30 novembre 2005 (2005-11-30), pages 1-6, XP010922196, ISBN: 978-0-7803-9419-3 * page 3 - page 5 * ----- | 2,4 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Munich | 3 avril 2012 | Purdie, Douglas |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 11 19 4786

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

03-04-2012

| Document brevet cité au rapport de recherche | | Date de publication | Membre(s) de la famille de brevet(s) | | Date de publication |
|---|---|---|---|---|---|
| JP 2002131262 | A | 09-05-2002 | AUCUN | | |
| US 5882937 | A | 16-03-1999 | AUCUN | | |
| EP 1338891 | A1 | 27-08-2003 | CN | 1439874 A | 03-09-2003 |
| | | | EP | 1338891 A1 | 27-08-2003 |
| | | | JP | 3820163 B2 | 13-09-2006 |
| | | | JP | 2003247965 A | 05-09-2003 |
| | | | US | 2003159947 A1 | 28-08-2003 |

EPO FORM P0460

Pour tout renseignement concernant cette annexe : voir Journal Officiel de l'Office européen des brevets, No.12/82